# EUROPÄISCHE PATENTANMELDUNG

(11) **EP 2 711 032 A2**
(43) Veröffentlichungstag der Anmeldung: **26.03.2014**
(21) Anmeldenummer: 13176546.3
(22) Anmeldetag: 15.07.2013
(51) Int. Cl.: A61L 27/46, A61L 24/00

(54) **Pastenförmiger Knochenzement**

(30) Priorität: 25.07.2012 DE 102012014702
(71) Anmelder: Heraeus Medical GmbH, 61273 Wehrheim (DE)
(72) Erfinder: Vogt, Sebastian, 99092 Erfurt (DE)
(74) Vertreter: Panten, Kirsten

(57) **Zusammenfassung**

Die vorliegende Erfindung betrifft eine Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikuläres anorganisches Calciumsalz ist, welches die Eigenschaften i) und ii) aufweist:
i) mindestens 90 Gew.-% des partikulären anorganischen Calciumsalzes besitzen eine durch Siebanalyse bestimmte Partikelgröße von weniger als 63 µm;
ii) das partikuläre anorganische Calciumsalz weist bei 20°C eine Löslichkeit in Wasser von weniger als 8,5 g pro Liter auf.

Die Erfindung betrifft auch einen Kit, die Verwendung einer Paste oder einer Paste, hergestellt aus dem erfindungsgemäßen Kit, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie, sowie ein Formkörper.

## Beschreibung

Die vorliegende Erfindung betrifft eine Paste, einen Kit, die Verwendung einer Paste oder einer Paste, hergestellt aus einem Kit, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie, sowie einen Formkörper.

Konventionelle Polymethylmethacrylat-Knochenzemente (PMMA-Knochenze-mente) sind seit Jahrzehnten bekannt und gehen auf die grundlegenden Arbeiten von Sir Charnley zurück (Charnley, J.: "Anchorage of the femoral head prosthesis of the shaft of the femur"; J. Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, (ii) einen Röntgenopaker und (iii) einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist.

Der wesentliche Nachteil der bisherigen PMMA-Knochenzemente für den medizinischen Anwender besteht darin, dass der Anwender die flüssige Monomerkomponente mit der Pulverkomponente unmittelbar vor der Zementapplikation in einem Mischsystem oder in Tiegeln vermischen muss. Dabei können leicht Mischungsfehler auftreten, welche die Zementqualität negativ beeinflussen. Ferner muss das Vermischen der Komponenten zügig erfolgen. Dabei kommt es darauf an, dass das gesamte Zementpulver ohne Klumpenbildung mit der Monomerkomponente vermischt wird und das beim Vermischungsvorgang der Eintrag von Luftblasen vermieden wird. Bei der Verwendung von Vakuummischsystemen wird im Gegensatz zur Handanmischung die Bildung von Luftblasen im Zementteig weitgehend verhindert. Beispiele für Mischsysteme sind in den Schriften US 4,015,945, EP-A-0 674 888 und JP-A-2003181270 offenbart. Vakuummischsysteme machen jedoch eine zusätzliche Vakuumpumpe erforderlich und sind daher relativ kostenintensiv. Nach der Vermischung der Monomerkomponente mit der Pulverkomponente muss ferner je nach Zementtyp eine gewisse Zeit gewartet werden, bis der Zementteig klebfrei ist und appliziert werden kann. Aufgrund der vielen Fehlermöglichkeiten beim Anmischen von konventionellen PMMA-Knochenzementen wird zudem entsprechend geschultes Personal benötigt. Die entsprechenden Schulungen sind mit einem erheblichen Kostenaufwand verbunden. Weiterhin kommt es bei der Vermischung der flüssigen Monomerkomponente mit der Pulverkomponente zu einer Belastung der Anwender durch Monomerdämpfe und durch Freisetzung von pulverförmigen Zementpartikeln.

Als Alternative zu den konventionellen Pulver-Flüssigkeits-Polymethylmethacrylatknochenzementen wurden pastenförmige Polymethylmethacrylatknochen-zemente in den Offenlegungsschriften DE-A-10 2007 052 116, DE-A-10 2007 050 762 und DE-A-10 2007 050 763 beschrieben, welche ein radikalisch polymerisierbares Methacrylat-Monomer, ein in diesem Methacrylat-Monomer lösliches Polymer und - ein in diesem Methacrylat-Monomer unlösliches partikuläres Polymer beinhalten. Solche pastenförmigen Polymethylmethacrylat-Knochenzemente können als Einkomponenten-System (in diesem Fall beinhaltet die Paste alle zur Aushärtung notwendigen Komponenten, insbesondere einen aktivierbaren radikalischen Initiator, z. B. einen Photoinitiator oder ein Photoiniatorsystem) oder als Zweikomponenten-System (in diesem Fall umfasst das System zwei vorgemischte, lagerstabile Pasten, von denen eine einen radikalischen Polymerisationsinitiator und die andere einen Polymerisationsaktivator aufweist) vorliegen. Im Falle der Zweikomponenten-Systeme wird weiterhin zwischen einem "symmetrischen System" (in diesem Fall beinhalten beide Pasten ein in dem Methacrylat-Monomer unlösliches partikuläres Polymer) und "unsymmetrischen Systemen" (in diesem Fall beinhaltet nur eine der beiden Pasten ein in dem Methacrylat-Monomer unlösliches partikuläres Polymer) unterschieden.

Der aus den vorstehen beschriebenen Pasten hergestellte Knochenzement weist als Folge der gewählten Zusammensetzung eine ausreichend hohe Viskosität und Kohäsion auf, um dem Blutungsdruck bis zur Aushärtung standzuhalten. Durch die fortschreitende Polymerisation härtet die Paste unter Verbrauch der Methacrylat-Monomere aus.

Bei den in DE-A-10 2007 052 116, DE-A-10 2007 050 762 und DE-A-10 2007 050 763 offenbarten pastenförmigen Polymethylmethacrylat-Knochenzementen sind neben wenigstens einem radikalisch polymerisierbaren Monomer und mindestens einem darin gelösten Polymer in dem Monomer unlösliche Polymerpartikel enthalten. Diese unlöslichen Polymerpartikel stellen einen Füllstoff dar. Dieser beeinflusst die Viskosität der Zementpasten erheblich. Die Polymerpartikel sind essentiell für die Verarbeitungseigenschaften, damit die Zementpasten während der Applikationsphase bei der Formgebung möglichst nur eine minimale elastische Rückstellbewegung zeigen. Dadurch lassen sich die Zementpasten in der Verarbeitungsphase in beliebige Gestalt formen, wie es von konventionellen Polymethylmethacrylat-Knochenzementen, die auf der Vermischung von Polymerpulver und Monomerflüssigkeit beruhen, allgemein bekannt ist.

In Methacrylatmonomeren unlösliche, vernetzte Polymerpartikel sind in der Herstellung relativ aufwendig und daher teuer. Daher ist es wünschenswert, ein alternatives, kostengünstiges, partikuläres Material zu finden, das nach Zusatz zu Gemischen aus Methacrylatmonomeren und darin gelösten Polymeren, in ähnlicher Weise wie vernetzte Polymerpartikel, Pasten ergibt, die nach Formgebung nur ein minimales elastisches Rückstellvermögen zeigen.

Problematisch ist jedoch, dass bisher verwendeten vernetzten Polymerpartikel auch einen Beitrag zur mechanischen Stabilität der ausgehärteten pastenförmigen Zemente lieferten. Es kommt deshalb darauf an, einen alternativen Füllstoff zu finden, der einerseits die erforderlichen Verarbeitungseigenschaften der Pasten gewährleistet und andererseits die mechanischen Parameter der ausgehärteten Zemente nicht negativ beeinflusst, so dass die Anforderungen der ISO 5833 hinsichtlich der mechanischen Stabilität erfüllt werden.

Der vorliegenden Erfindung lag die Aufgabe zugrunde, die sich aus dem Stand der Technik ergebenden Nachteile im Zusammenhang mit auf Pasten basierenden Knochenzement-Systemen, insbesondere im Zusammenhang mit den vorstehend beschriebenen Zweikomponenten-Systemen, zu überwinden.

Insbesondere lag der vorliegenden Erfindung die Aufgabe zugrunde, eine Knochenzementpaste, insbesondere eine auf einem Zweikomponenten-System basierenden Knochenzementpaste bereitzustellen, die im Vergleich zu den aus den Stand der Technik bekannten Knochenzementpasten aus kostengünstigeren Ausgangsmaterialien hergestellt werden kann, die aber dennoch die gleichen Verarbeitungseigenschaften aufweist wie die Pasten des Standes der Technik.

Einen Beitrag zur Lösung der eingangs genannten Aufgaben leistet eine Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikuläres anorganische Calciumsalz ist, welches die Eigenschaften i) und ii) aufweist:
i) mindestens 90 Gew.-%, besonders bevorzugt mindestens 95 Gew.-% und am meisten bevorzugt 100 Gew.-% des partikulären anorganischen Calciumsalzes besitzen eine durch Siebanalyse bestimmte Partikelgröße von weniger als 63 µm, besonders bevorzugt von weniger als 20 µm und am meisten bevorzugt von weniger als 10 µm;
ii) das partikuläre anorganische Calciumsalz weist bei 20°C eine Löslichkeit in Wasser von weniger als 8,5 g pro Liter, besonders bevorzugt von weniger als 5 g pro Liter und am meisten bevorzugt von weniger als 3 g pro Liter auf.

Die Erfindung basiert auf dem gegenüber den bisher bekannten pastenförmigen Polymethylmethacrylat-Knochenzementen überraschenden Befund, dass sich gut formbare, modellierbare Knochenzementpasten unter Verwendung von partikulären, anorganischen Calciumsalzen der Siebfraktion kleiner 63 µm (gemäß DIN 66165-1/-2) herstellen lassen. Überraschend ist, dass die bisher üblichen, in Methacrylat-Monomeren unlöslichen, vernetzten Polymerpartikel ganz bzw. teilweise durch partikuläre, anorganische Calciumsalze ersetzt werden können. Anorganische Calciumsalze sind deutlich preiswerter als vernetzte Polymerpartikel und können dadurch wirtschaftlich vorteilhaft zur Herstellung von pastenförmigen Polymethylmethacrylat-Knochenzementen verwendet werden. Es wurde überraschend gefunden, dass besonders Calciumcarbonat der Siebfraktion kleiner 63 µm als Füllstoff besonders gut geeignet ist. Anorganische Calciumsalze wie beispielsweise Calciumcarbonat haben eine relativ geringe Härte. So ist Calciumcarbonat (Calcit) durch eine Härte nach Mohs von 2 gekennzeichnet. Auf Grund der geringen Härte war zu erwarten, dass durch Inkorporation von partikulären, anorganischen Calciumsalzen Polymethylmethacrylat-Pasten die ausgehärteten Zementpasten nicht den Anforderungen der ISO 5833 hinsichtlich der 4-Punkt-Biegefestigkeit, des Biegemoduls und der Druckfestigkeit genügen werden. Daher war es umso überraschender, dass es gelang, trotz der Verwendung von partikulären, anorganischen Calciumsalzen anstelle von vernetzten Polymerpartikeln Zementpasten herzustellen, die nach erfolgter Aushärtung die mechanischen Anforderungen der IS05833 erfüllen.

Grundsätzlich kann die erfindungsgemäße Paste ein vorstehend beschriebenes Einkomponenten-System sein oder durch Vermischen der beiden Pasten eines vorstehend beschriebenen Zweikomponenten-Systems erhalten werden.

Die erfindungsgemäße Paste beinhaltet als eine Komponente wenigstens ein radikalisch polymerisierbares Monomer, wobei es sich vorzugsweise um ein Methacrylat-Monomer, insbesondere um ein Methacrylat-Monomer handelt, welches bei einer Temperatur von 25°C und einem Druck von 1.013 hPa flüssig ist.

Vorzugsweise handelt es sich bei dem radikalisch polymerisierbaren Monomer nicht um einen von Bisphenol A abgeleiteten Methacrylsäureester.

Das Methacrylat-Monomer ist vorzugsweise ein Methacrylsäureester. Vorzugsweise handelt es sich bei dem Methacrylsäureester um einen monofunktionellen Methacrylsäureester. Dieser ist vorzugsweise hydrophob. Durch die Verwendung von hydrophoben monofunktionellen Methacrylsäureestern kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Das erfindungsgemäß eingesetzte radikalisch polymerisierbare Monomer weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer zeichnet sich vorzugsweise dadurch aus, dass eine wässrige Lösung des radikalisch polymerisierbaren Monomers einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Gemäß einer besonders bevorzugten Ausführungsform handelt es sich bei dem Methacrylat-Monomer um Methacrylsäuremethylester, Methacrylsäureethylester oder um eine Mischung aus diesen beiden Monomeren.

Vorzugsweise beinhaltet die erfindungsgemäße Paste das radikalisch polymerisierbare Monomer in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der erfindungsgemäßen Paste.

Als weitere Komponente beinhaltet die erfindungsgemäße Paste wenigstens ein in dem wenigstens einen radikalisch polymerisierbaren Monomer lösliches Polymer. Ein Polymer ist erfindungsgemäß in dem polymerisierbaren Monomer löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse von wenigstens 150.000 g/mol. Beispielsweise kann es sich bei dem löslichen Polymer um ein Polymer oder Copolymer eines Methacrylsäureesters handeln. Gemäß einer besonders bevorzugten Ausführungsform ist das wenigstens eine lösliche Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Die Menge des in dem radikalisch polymerisierbaren Monomer löslichen Polymers in der erfindungsgemäßen Paste liegt üblicherweise in einem Bereich von 1 bis 85 Gew.-%, bezogen auf das Gesamtgewicht der erfindungsgemäßen Paste.

Weiterhin beinhaltet die erfindungsgemäße Paste wenigstens ein in dem wenigstens einen radikalisch polymerisierbaren Monomer schwer- oder unlösliches partikuläres anorganisches Calciumsalz mit den vorstehend definierten Eigenschaften i) und ii) als Füllstoff.

Gemäß einer bevorzugten Ausgestaltung der erfindungsgemäßen Paste ist das partikuläre anorganische Calciumsalz ausgewählt aus der Gruppe bestehend aus Calciumcarbonat, Dolomit, Calciumsulfat-Dihydrat, α-Tricalciumphosphat, β-Tricalcium-phosphat, Hydroxylapatit, Octacalciumphosphat, amorphes Calciumphosphat, Fluorapatit, Chlorapatit, Carbonatapatit und einer Mischung aus mindestens zwei davon. Unter diesen besonders bevorzugt sind partikuläre anorganische Calciumsalze ausgewählt aus der Gruppe bestehend aus Calciumcarbonat, Dolomit, Calciumsulfat-Dihydrat, β-Tricalciumphosphat, Hydroxylapatit, und einer Mischung aus mindestens zwei davon, wobei Calciumcarbonat als partikuläres anorganisches Calciumsalz am meisten bevorzugt ist.

Die Menge des partikulären anorganischen Calciumsalzes in der erfindungsgemäßen Paste liegt üblicherweise in einem Bereich von 0,5 bis 25 Gew.-%, besonders bevorzugt von 1 bis 20 Gew.-% und am meisten bevorzugt in einem Bereich von 5 bis 15 Gew.-%, jeweils bezogen auf das Gesamtgewicht der Paste. Gegebenenfalls kann die erfindungsgemäße Paste neben dem vorstehend beschriebenen partikulären anorganischen Calciumsalz auch noch bestimmte Mengen eines anderen Füllstoffes, beispielsweise die aus dem Stand der Technik bekannten, vernetzten Polymerpartikel, beinhalten, wobei in diesem Falle das Gewichtsverhältnis von partikulärem anorganischem Calciumsalz zu den vernetzten Polymerpartikeln vorzugsweise mindestens 1 : 15 (also mindestens etwa 6 Gew.-% partikuläres anorganisches Calciumsalz bezogen auf die Gesamtmenge an Füllstoff), ganz besonders bevorzugt mindestens 1 : 1 (also mindestens 50 Gew.-% partikuläres anorganisches Calciumsalz bezogen auf die Gesamtmenge an Füllstoff) beträgt.

Vorzugsweise ist die erfindungsgemäße Paste spätestens 15 Minuten nach ihrer Herstellung klebfrei nach ISO 5833.

Die erfindungsgemäße Paste kann weiterhin wenigstens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder wenigstens einen Polymerisations-initiator, wenigstens einen Polymerisationsbeschleuniger und gegebenenfalls wenigstens einen Co-Polymerisationsbeschleuniger beinhalten.

Im Falle eines Einkomponenten-Systems handelt es sich bei dem Polymerisationsinitiator vorzugsweise um einen aktivierbaren Polymerisationsinitiator, z. B. um einen in der Paste gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle. Auch kann im Falle eines Einkomponenten-Systems die erfindungsgemäße Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Im Falle einer erfindungsgemäßen Paste, welche durch die Kombination zweier Pasten eines Zweikomponenten-Systems erhalten wurde, beinhaltet diese Paste vorzugsweise wenigstens einen Polymerisationsinitiator (der in der einen Paste des Zweikomponenten-Systems enthalten war) und wenigstens einen Polymerisationsbeschleuniger (der in der anderen Paste des Zweikomponenten-Systems enthalten war).

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden erfindungsgemäß Verbindungen verstanden, die wenigstens eine Peroxogruppe (-O-O-)enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Gemäß einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amyl-hydroperoxid, Di-Isopropylbenzen-mono-hydroperoxid und einer Mischung aus mindestens zwei davon besteht.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten-und 1,3,5-trisubstituierten Barbituraten. Gemäß einer besonderen Ausgestaltung der erfindungsgemäßen Paste ist das Barbitursäurederivat ausgewählt ist aus der Gruppe bestehend aus 1,5-disubstituierten Barbituraten-und 1,3,5-trisubstituierten Barbituraten.

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf. Erfindungsgemäß bevorzugt sind Barbiturate, die an Position 1 und an Position 5 jeweils einen Substituenten oder an den Positionen 1, 3 und 5 jeweils einen Substituenten -tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt-

Erfindungsgemäß bevorzugte Schwermetallverbindungen sind ausgewählt aus der Gruppe bestehend Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform der erfindungsgemäßen Paste ist der Polymerisationsbeschleuniger ausgewählt aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammo-niumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäurediimid und einer Mischung aus mindestens zwei davon.

Eine weitere vorteilhafte Ausgestaltung der Erfindung besteht in Verwendung von Kombinationen aus Schwermetallsalzen und mindestens einem Vertreter aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei sind Zweifachkombinationen und auch Dreifachkombinationen von unterschiedlichen Polymerisationsbeschleunigern im Rahmen der Erfindung.

Eine vorteilhafte Ausgestaltung der Erfindung besteht darin, dass gegebenenfalls wenigstens ein Co-Polymerisationsbeschleuniger in der erfindungsgemäßen Paste enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethyl-anilin, 1,8-Diazabicyclo[5.4.0-]undec-7-en und 1,5-Diazabicyclo(4.3.0)-non-5-en als Co-Akzeleratoren bevorzugt sind.

Die erfindungsgemäße Paste kann den Polymerisationsinitiator, den Polymerisationsbeschleuniger, den Co-Polymerisationsbeschleuniger oder den Polymerisationsinitiator, den Polymerisationsbeschleuniger und den Co-Polymerisationsbeschleuniger in einer (Gesamt)Menge von bis zu 10 Gew.-%, bezogen auf das Gesamtgewicht der erfindungsgemäßen Paste, beinhalten.

Die erfindungsgemäße Paste kann neben den vorstehend genannten Komponenten weitere Bestandteile beinhalten.

Gemäß einer bevorzugten Ausführungsform der erfindungsgemäßen Paste kann diese wenigstens ein Röntgenopaker beinhalten. Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentration an Röntgenopaker, insbesondere die Konzentration an Zirkoniumdioxid, in der erfindungsgemäßen Paste kann in beispielsweise in einem Bereich von 3 bis 30 Gew.-% liegen.

Gemäß einer weiteren bevorzugten Ausführungsform kann die erfindungsgemäße Paste wenigstens einen Farbstoff beinhalten. Der Farbstoff kann ein fachüblicher Farbstoff und vorzugsweise ein Lebensmittelfarbstoff sein. Ferner kann der Farbstoff in dem wenigstens einen radikalisch polymerisierbaren Monomer löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform kann die erfindungsgemäße Paste wenigstens einen pharmazeutischen Wirkstoff beinhalten. Der wenigstens eine pharmazeutische Wirkstoff kann in der erfindungsgemäßen Paste in gelöster oder suspendierter Form enthalten sein. Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum. Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmern, Carbapenemen, cyclischen Lipopeptiden, Glycylcyclinen, Oxazolidonen und Polypeptidantibiotika besteht. Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht. Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycin-hydrochlorid, Lincosaminhydrochlorid und Moxifloxacin besteht. Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht. Das wenigstens eine Hormon ist vorzugsweise aus der Gruppe ausgewählt, die aus Serotonin, Somatotropin, Testosteron und Östrogen besteht. Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht. Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht. Das wenigstens eine Bisphosphonat ist vorzugsweise aus der Gruppe ausgewählt, die aus Zoledronat und Aledronat besteht.

Gemäß einer weiteren bevorzugten Ausführungsform kann die erfindungsgemäße Paste wenigstens ein biokompatibles Elastomer beinhalten. Das biokompatible Elastomer ist vorzugsweise partikulär. Vorzugsweise ist das biokompatible Elastomer in dem wenigstens einen radikalisch polymerisierbaren Monomer löslich. Als besonders geeignet hat sich die Verwendung von Polybutadien als biokompatibles Elastomer erwiesen.

Gemäß einer weiteren bevorzugten Ausführungsform kann die erfindungsgemäße Paste wenigstens ein Monomer mit Haftgruppen beinhalten. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform kann die erfindungsgemäße Pasten wenigstens einen Stabilisator beinhalten. Der Stabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemäßen Paste enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Einen Beitrag zur Lösung der eingangs genannten Aufgabe leistet auch ein Kit, aufweisend eine Paste A und eine Paste B,
wobei
(a) Paste A
   (a1) wenigstens ein radikalisch polymerisierbares Monomer,
   (a2) wenigstens ein in (a1) lösliches Polymer, und
   (a3) wenigstens einen Polymerisationsinitiator beinhaltet;
(b) Paste B
   (b1) wenigstens ein radikalisch polymerisierbares Monomer,
   (b2) wenigstens ein in (b1) lösliches Polymer, und
   (b3) wenigstens einen Polymerisationsbeschleuniger beinhaltet;
und wobei mindestens eine der Pasten A und B als Komponente (a4) bzw. (b4) wenigstens einen in (a1) bzw. (b1) schwer- oder unlöslichen Füllstoff, beinhaltet, wobei der Füllstoff ein partikuläres anorganisches Calciumsalz ist, welches die Eigenschaften i) und ii) aufweist:
i) mindestens 90 Gew.-%, besonders bevorzugt mindestens 95 Gew.-% und am meisten bevorzugt 100 Gew.-% des partikulären anorganischen Calciumsalzes besitzen eine durch Siebanalyse bestimmte Partikelgröße von weniger als 63 µm, besonders bevorzugt von weniger als 20 µm und am meisten bevorzugt von weniger als 10 µm;
ii) das partikuläre anorganische Calciumsalz weist bei 20°C eine Löslichkeit in Wasser von weniger als 8,5 g pro Liter, besonders bevorzugt von weniger als 5 g pro Liter und am meisten bevorzugt von weniger als 3 g pro Liter auf.

Erfindungsgemäß wird unter Kit ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Vorzugsweise ist der Kit als Vorrichtung zur Herstellung von Knochenzement ausgestaltet, die einen ersten Behälter und einen zweiten Behälter aufweist, wobei der erste Behälter die Paste A und der zweite Behälter die Paste aufweist, wobei wenigstens einer der Behälter zu öffnen ist, um nach dem Öffnen ein Vermischen der Pasten A und B zu gestatten, und eine Mischeinheit zum Vermischen der Pasten A und B.

Als radikalisch polymerisierbares Monomer (a1) bzw. (b1), als in (a1) bzw. (b1) lösliches Polymer (a2) bzw. (b2), als Polymerisationsinitiator (a3), als Polymerisationsbeschleuniger (b3) und als partikuläres anorganisches Calciumsalz (a4) bzw. (b4) sind diejenigen Komponenten bevorzugt, die bereits eingangs im Zusammenhang mit der erfindungsgemäßen Paste als bevorzugtes radikalisch polymerisierbares Monomer, als in diesem Monomer lösliches Polymer, als Polymerisationsinitiator, als Polymerisationsbeschleuniger und als partikuläres anorganisches Calciumsalz beschrieben wurden.

Vorzugsweise beinhalten die Paste A und die Paste B das wenigstens eine radikalisch polymerisierbare Monomer (a1) bzw. (b1) in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste A bzw. der Paste B.

Vorzugsweise beinhaltet die Paste A den Polymerisationsinitiator (a3) in einer Menge ein einem Bereich von 0,01 bis 10 Gew.-%, mehr bevorzugt in einem Bereich von 0,01 bis 8 Gew.-% und noch mehr bevorzugt in einem Bereich von 0,01 bis 5 Gew.-%, jeweils bezogen auf das Gesamtgewicht der Paste A.

Sofern es sich bei dem Polymerisationsbeschleuniger (b3) um eine Schwermetallverbindung handelt, die aus der Gruppe bestehend aus Schwermetallsalzen und Schwermetallkomplexen ausgewählt ist, insbesondere jedoch um eine Schwermetallverbindung, die ausgewählt ist aus der Gruppe bestehend aus Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon, so beinhaltet die Paste B einen solchen Polymerisationsbeschleuniger (b3) vorzugsweise in einer Menge in einem Bereich von 0,0005 bis 0,5 Gew.-%, bezogen auf das Gesamtgewicht der Paste B.

Sofern es sich bei dem Polymerisationsbeschleuniger (b3) um eine Verbindung ausgewählt ist aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en, 1,5-Diazabicyclo(4.3.0)non-5-en, Phthalimid, Maleimid, Succinimid, Pyromelithsäurediimid und eine Mischung aus mindestens zwei davon handelt, so beinhaltet die Paste B einen solchen Polymerisationsbeschleuniger (b3) vorzugsweise in einer Menge in einem Bereich von 0,1 bis 10 Gew.-%, bezogen auf das Gesamtgewicht der Paste B.

Insbesondere die Paste A kann weiterein als Komponente (a5) den vorstehend beschriebenen Co-Polymerisationsbeschleuniger beinhalten, bei dem es sich vorzugsweise um eine Verbindung ausgewählt ist aus der Gruppe bestehend aus N,N-Dimethyl-p-toluidin, N,N-Bishydroxyethyl-p-toluidin, N,N-Dimethyl-anilin, 1,8-Diazabicyclo[5.4.0]undec-7-en, 1,5-Diazabicyclo(4.3.0)non-5-en und einer Mischung aus mindestens zwei davon handelt. In diesem Zusammenhang ist es bevorzugt, dass die Paste A den wenigstens einen Co-Polymerisationsbeschleuniger (a5) in einer Menge in einem Bereich von 0,1 bis 10 Gew.-%, bezogen auf das Gesamtgewicht der A, beinhaltet.

Sofern eine der beiden Pasten des erfindungsgemäßen Kits den schwer- oder unlöslichen Füllstoff beinhaltet und die andere Paste überhaupt keinen schwer- oder unlöslichen Füllstoff oder einen schwer- oder unlöslichen Füllstoff in einer im Vergleich zur Menge in der anderen Paste vernachlässigbaren Menge, so handelt es sich um einen sogenannten "asymmetrischen" Kit. Ist hingegen der schwer- oder unlösliche Füllstoff in beiden Pasten in etwa vergleichbarer Menge vorhanden, so handelt es sich um einen sogenannten "symmetrischen" Kit.

Weiterhin können in den Pasten A und/oder B außer den vorstehend beschriebenen Komponenten auch weitere Zusatzstoffe enthalten sein, wie etwa Röntgenopaker, Farbstoffe, pharmazeutische Wirkstoffe, biokompatible Elastomere, Monomere mit Haftgruppen oder Stabilisatoren, wobei auch hier diejenigen Komponenten bevorzugt sind, die bereits eingangs im Zusammenhang mit der erfindungsgemäßen Paste als bevorzugte Röntgenopaker, Farbstoffe, pharmazeutische Wirkstoffe, biokompatible Elastomere, Monomere mit Haftgruppen und Stabilisatoren beschrieben wurden.

Gemäß einer ersten besonderen Ausgestaltung des erfindungsgemäßen Kits handelt es sich bei dem Kit um einen "asymmetrischen" Kit. In diesem Zusammenhang ist es bevorzugt, dass die Paste A 20 bis 70 Gew.-%, besonders bevorzugt 25 bis 60 Gew.-%, noch mehr bevorzugt 30 bis 55 Gew.-% und am meisten bevorzugt 34 bis 47 Gew.-%, jeweils bezogen auf das Gesamtgewicht der Paste A, des in (a1) unlöslichen Füllstoffs (a4) und Paste B weniger als 5 Gew.-%, besonders bevorzugt weniger als 1 Gew.-%, noch mehr bevorzugt weniger als 0,1 Gew.-% und darüber hinaus bevorzugt weniger als 0,01 Gew.-%, jeweils bezogen auf das Gesamtgewicht der Paste B, des in (b1) unlöslichen Füllstoffs (b4) beinhaltet, wobei es am meisten bevorzugt ist, dass die Paste B überhaupt keinen in (b1) unlöslichen Füllstoff (b4) beinhaltet.

Weiterhin ist es im Zusammenhang mit dieser ersten besonderen Ausgestaltung des erfindungsgemäßen Kits bevorzugt, dass Paste A das in (a1) lösliche Polymer (a2) in einer Menge in einem Bereich von 1 bis 25 Gew.-%, besonders bevorzugt in einem Bereich von 2 bis 20 Gew.-%, noch mehr bevorzugt in einem Bereich von 2 bis 18 Gew.-% und am meisten bevorzugt in einem Bereich von 3 bis 16 Gew.-%, jeweils bezogen auf das Gesamtgewicht der Paste A, und Paste B ein in (b1) lösliches Polymer (b2) in einer Menge in einem Bereich von 25 bis 85 Gew.-%, besonders bevorzugt in einem Bereich von 35 bis 85 Gew.-%, noch mehr bevorzugt in einem Bereich von 35 bis 80 Gew.-% und am meisten bevorzugt in einem Bereich von 35 bis 75 Gew.-%, jeweils bezogen auf das Gesamtgewicht der Paste B, beinhaltet.

Weiterhin ist es im Zusammenhang mit dieser ersten besonderen Ausgestaltung des erfindungsgemäßen Kits bevorzugt, dass das Gewichtsverhältnis von in (b1) unlöslichem Füllstoff (b4) zu dem wenigstens einen in (b1) löslichen Polymer (b2) höchstens 0,2, besonders bevorzugt höchstens 0,15, noch mehr bevorzugt höchstens 0,1, nicht mehr bevorzugt höchstens 0,05, besonders bevorzugt höchstens 0,02 und ganz besonders bevorzugt 0 beträgt.

Gemäß einer zweiten besonderen Ausgestaltung des erfindungsgemäßen Kits handelt es sich bei dem Kit um einen "symmetrischen" Kit. In diesem Zusammenhang ist es bevorzugt, dass die Paste A 15 bis 85 Gew.-%, besonders bevorzugt 15 bis 80 Gew.-% und noch mehr bevorzugt 20 bis 75 Gew.-%, jeweils bezogen auf das Gesamtgewicht der Paste A, des in (a1) unlöslichen Füllstoffs (a4) und Paste B 15 bis 85 Gew.-%, besonders bevorzugt 15 bis 80 Gew.-% und noch mehr bevorzugt 20 bis 75 Gew.-%, jeweils bezogen auf das Gesamtgewicht der Paste B, des in (b1) unlöslichen Füllstoffs (b4) beinhaltet.

Weiterhin ist es im Zusammenhang mit dieser zweiten besonderen Ausgestaltung des erfindungsgemäßen Kits bevorzugt, dass Paste A ein in (a1) lösliches Polymer (a2) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, jeweils bezogen auf das Gesamtgewicht der Paste A, und/oder Paste B ein in (b1) lösliches Polymer (b2) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, bezogen auf das Gesamtgewicht der Paste B, beinhaltet.

Erfindungsgemäß dient die erfindungsgemäße Paste bzw. der erfindungsgemäße Kit, der wenigstens die Pasten A und B enthält, zur Herstellung von Knochenzement.

Im Falle des Kits werden dazu die wenigstens zwei Pasten A und B miteinander vermischt, wobei wiederum eine Paste, Paste C, erhalten wird. Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B. Das Mischen kann mit üblichen Mischgeräten, beispielsweise einem statischen Mischer oder einem dynamischen Mischer erfolgen.

Nach dem Vermischen der Pasten des Kits ist die letztlich erhaltene Paste C (die der eingangs genannten erfindungsgemäßen Paste entspricht) spätestens nach 15 Minuten nach der Norm ISO 5833 klebfrei.

Der aus der erfindungsgemäßen Paste bzw. der aus Paste C durch Aushärtung entstehende Knochenzement erreicht etwa sechs bis acht Minuten nach dem Vermischen der im Kit enthaltenen Pasten eine hohe Festigkeit.

Die erfindungsgemäße Paste bzw. die Paste C, hergestellt aus dem erfindungsgemäßen Kit, kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacern und zur Herstellung von Trägermaterialien für die lokale Antibiotika-Therapie verwendet werden.

Unter dem Begriff *"Spacer"* werden dabei erfindungsgemäß Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus der erfindungsgemäßen Paste bzw. dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.

Die vorstehend beschriebenen erfindungsgemäßen Verwendungen von Knochenzement sind aus der Literatur bekannt und dort mannigfach beschrieben.

Einen Beitrag zur Lösung der eingangs genannten Aufgaben leistet auch ein Formkörper, erhältlich durch die Polymerisation einer Paste, erhältlich durch das Vermischen der Paste A und der Paste B des erfindungsgemäßen Kits, oder durch die Polymerisation einer erfindungsgemäßen Paste. Formkörper im Sinne der vorliegenden Erfindung können jedwede dreidimensionalen Körper sein, insbesondere die vorstehend beschriebenen "Spacer".

Die Erfindung wird durch die nachstehend beschriebenen Beispiele erläutert, die jedoch die Erfindung nicht einschränken.

### AUSFÜHRUNGSBEISPIELE

Die Pasten A der Beispiele A1-8 wurden durch einfaches Vermischen der Komponenten hergestellt. Die gebildeten Pasten wurden dann über Nacht bei Raumtemperatur gelagert.

**Paste A**

| | Zusammensetzung der Pasten A | | | | | | | | | |
|---|---|---|---|---|---|---|---|---|---|---|
| Beispiel-Nr. | CH [mg] | BH [g] | DM [g] | EG [g] | MA [g] | MMA [g] | PL [g] | CA [g] | ZrO₂ [g] | Stab [mg] |
| A1 | 50 | 1,40 | 0,60 | 0,1 | 0,4 | 1,70 | 13,0 | 7,0 | 4,8 | 20 |
| A2 | 50 | 1,40 | 0,60 | 0,1 | 0,4 | 1,70 | 13,0 | 9,0 | 4,8 | 20 |
| A3 | 50 | 1,40 | 0,60 | 0,1 | 0,4 | 1,70 | 13,0 | 11,0 | 4,8 | 20 |
| A4 | 50 | 1,40 | 0,60 | 0,1 | 0,4 | 1,70 | 13,0 | 13,0 | 4,8 | 20 |
| A5 | 50 | 1,40 | 0,60 | 0,1 | 0,4 | 1,70 | 13,0 | 14,0 | 4,8 | 20 |
| A6 | 50 | 1,40 | 0,60 | 0,1 | 0,4 | 1,70 | 13,0 | 16,0 | 4,8 | 20 |
| A7 | 50 | 1,40 | 0,60 | 0,1 | 0,4 | 1,70 | 13,0 | 14,0 | 4,8 | 20 |
| A8 | 50 | 1,40 | 0,60 | 0,1 | 0,4 | 1,70 | 13,0 | 16,0 | 4,8 | 20 |

| | | | | | | | | | | |
|---|---|---|---|---|---|---|---|---|---|---|
| CH: Cumolhydroperoxid BH: N,N-Bis-(2-hydroxyethyl)-p-toluidin DM: N,N-Dimethyl-p-toluidin EG: Ethylenglykoldimethacrylat MA: Methacrylamid MMA: Methylmethacrylat PL: lineares Poly(methylmethacrylat-co-methylacrylat) Mw < 500.000 g/mol CA: Calciumcarbonat (Siebfraktion kleiner 63 µm) ZrO₂: Zirkoniumdioxid Stab: 2,6-Di-t-butyl-4-methyl-phenol | | | | | | | | | | |

Die Pasten B der Beispiele B1-8 wurden ebenfalls durch einfaches Vermischen der Komponenten hergestellt. Die gebildeten Pasten wurden dann über Nacht bei Raumtemperatur gelagert.

**Paste B**

| | Zusammensetzung der Pasten B | | | | | | |
|---|---|---|---|---|---|---|---|
| Beispiel Nr. | SAC [g] | CuOct [mg] | MMA [g] | PL [g] | HM [g] | GS [g] | Stab [mg] |
| B1 | 1,00 | 55 | 21,20 | 17,50 | - | - | 15 |
| B2 | 1,00 | 55 | 21,20 | 17,50 | - | - | 15 |
| B3 | 1,00 | 55 | 21,20 | 17,50 | - | - | 15 |
| B4 | 1,00 | 55 | 21,20 | 17,50 | - | - | 15 |
| B5 | 1,00 | 40 | 21,20 | 17,50 | 0,17 | - | 15 |
| B6 | 1,00 | 45 | 21,20 | 17,50 | 0,17 | - | 15 |
| B7 | 1,00 | 40 | 21,20 | 17,50 | 0,17 | 1,26 | 15 |
| B8 | 1,00 | 45 | 21,20 | 17,50 | 0,17 | 1,26 | 15 |

| | | | | | | | |
|---|---|---|---|---|---|---|---|
| SAC: Saccharin CuOct: Kupfer(II)-2-ethylhexanoat MMA: Methylmethacrylat PL: lineares Poly(methylmethacrylat-co-methylacrylat), Mw < 500.000 g/mol HM: Methacrylsaäure-2-hydroxyethylester Stab: 2,6-Di-t-butyl-4-methyl-phenol GS: Gentamicinsulfat (Aktivitätskoeffizient AK= 621) | | | | | | | |

Die Pasten A und B der Beispiele A1-8 und B1-8 wurden im Gewichtverhältnis 1:1 miteinander gemischt. Es entstanden sofort klebfreie Pasten C, die eine Verarbeitungsphase ähnlich zu konventionellen hoch-viskosen Polymethylmethycrylat-Knochenzementen hatten. Die Verarbeitungsphase dauerte 4-6 Minuten.

Mit den aus den Pasten A und B der Beispiele 1-8 hergestellten gemischten Pasten C (Gewichtsverhältnis 1 zu 1 von Paste A zu Paste B) wurden streifenförmige Prüfkörper mit den Abmessungen (75 mm x 10 mm x 3,3 mm) für die Bestimmung der Biegefestigkeit und des Biegemoduls hergestellt und zylindrische Formkörper (Durchmesser 6 mm, Höhe 12 mm) für die Bestimmung der Druckfestigkeit angefertigt. Die Probekörper wurden dann 24 Stunden bei 23 ± 1 °C an der Luft gelagert. Danach wurden die 4-Punkt-Biegefestigkeit, das Biegemodul und die Druckfestigkeit der Prüfkörper mit einer Zwick-Universalprüfmaschine ermittelt.

| Pasten C | Zusammensetzung der Pasten C | 4-Punkt-Biegefestigkeit [MPa] | Biege-Modul [MPa] | DruckFestigkeit [MPa] |
|---|---|---|---|---|
| C1 | A1 + B1 | 63,6 ± 2,1 | 2777 ± 42 | 109,0 ± 3,3 |
| C2 | A2 + B2 | 67,2 ± 1,0 | 2796 ± 40 | 110,2 ± 5,8 |
| C3 | A3 + B3 | 62,7 ± 2,3 | 2713 ± 91 | 101,2 ± 3,2 |
| C4 | A4 + B4 | 60,0 ± 1,7 | 2583 ± 157 | 104,5 ± 3,7 |
| C5 | A5 + B5 | 57,5 ± 2,8 | 2667 ± 105 | 96,2 ± 3,5 |
| C6 | A6 + B6 | 54,9 ± 1,8 | 2523 ± 67 | 91,2 ± 3,5 |
| C7 | A7 + B7 | 57,5 ± 1,5 | 2567 ± 115 | 95,1 ± 1,9 |
| C8 | A8 + B8 | 54,0 ± 2,2 | 2400 ± 137 | 91,1 ± 2,4 |

Die ISO 5833 schreibt folgende Parameter vor: eine 4-Punkt-Biegefestigkeit von mindestens 50 MPa, ein Biegemodul von mindestens 1800 MPa und eine Druckfestigkeit von mindestens 70 MPa. Die Ergebnisse der Prüfung der 4-Punkt-Biegefestigkeit, des Biegemoduls und der Druckfestigkeit der ausgehärteten Pasten C1-8 zeigen, dass die Anforderungen der ISO 5833 hinsichtlich der mechanischen Festigkeit erfüllt werden.

Es wurden zusätzlich auch Pasten unter Verwendung von Bariumsulfat anstelle von Zirkoniumdioxid hergestellt. Diese Pasten hatten ein ähnliches Aushärtungsverhalten wie die aus den Pasten A1-8 und B1-8 hergestellten Pasten C1-8.

Außerdem wurden weitere Pasten B analog der Paste B8, jedoch mit einem Zusatz von jeweils 1,0 g Vancomycinhydrochlorid, Clindamycinhydrochlorid, Daptomycin und Octenidindihydrochlorid hergestellt. Nach Vermischung dieser Pasten B mit der Paste A1 im Gewichtsverhältnis von 1 zu 1 zeigten die gemischten Pasten C ein ähnliches Aushärtungsverhalten wie die Kombination der Paste A8 mit der Paste B8 im Gewichtsverhältnis von 1 zu 1.

Es wurden weiterhin auch Pasten A analog dem Beispiel A1 unter Verwendung von t-Butyl-hydroperoxid, t-Amyl-hydroperoxid und Dicumyl-peroxid anstelle von Cumol-hydroperoxid hergestellt. Nach Vermischung dieser Pasten A mit der Paste B1 im Gewichtsverhältnis von 1 zu 1 zeigten die gemischten Pasten ein ähnliches Verhalten wie die Kombination der Pasten A1 mit der Paste B1.

## Patentansprüche

1. Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikuläres anorganisches Calciumsalz ist, welches die Eigenschaften i) und ii) aufweist:
i) mindestens 90 Gew.-% des partikulären anorganischen Calciumsalzes besitzen eine durch Siebanalyse bestimmte Partikelgröße von weniger als 63 µm;
ii) das partikuläre anorganische Calciumsalz weist bei 20°C eine Löslichkeit in Wasser von weniger als 8,5 g pro Liter auf.

2. Paste nach Anspruch 1, wobei der Füllstoff die Eigenschaften i) und ii) aufweist:
i) mindestens 90 Gew.-% des partikulären anorganischen Calciumsalzes besitzen eine durch Siebanalyse bestimmte Partikelgröße von weniger als 20 µm;
ii) das partikuläre anorganische Calciumsalz weist bei 20°C eine Löslichkeit in Wasser von weniger als 5 g pro Liter auf.

3. Paste nach Anspruch 1 oder 2, wobei das partikuläre anorganische Calciumsalz ausgewählt ist aus der Gruppe bestehend aus Calciumcarbonat, Dolomit, Calciumsulfat-Dihydrat, α-Tricalciumphosphat, β-Tricalciumphosphat, Hydroxylapatit, Octacalciumphosphat, amorphes Calciumphosphat, Fluorapatit, Chlorapatit, Carbonatapatit und einer Mischung aus mindestens zwei davon.

4. Paste nach Anspruch 3, wobei das partikuläre anorganische Calciumsalz ausgewählt ist aus der Gruppe bestehend aus Calciumcarbonat, Dolomit, Calciumsulfat-Dihydrat, β-Tricalciumphosphat, Hydroxylapatit und einer Mischung aus mindestens zwei davon.

5. Paste nach Anspruch 4, wobei das partikuläre anorganische Calciumsalz Calciumcarbonat ist.

6. Paste nach einem der vorhergehenden Ansprüche, wobei die Paste 0,5 bis 25 Gew.-%, bezogen auf das Gesamtgewicht der Paste, des partikulären anorganischen Calciumsalzes beinhaltet.

7. Paste nach einem der vorhergehenden Ansprüche, wobei das radikalisch polymerisierbare Monomer ein Methacrylsäureester ist.

8. Paste nach einem der vorhergehenden Ansprüche, wobei die Paste zusätzlich wenigstens einen Polymerisationsinitiator, wenigstens einen Polymerisationsbeschleuniger, gegebenenfalls wenigstens einen Co-Polymerisationsbeschleuniger oder wenigstens einen Polymerisations-initiator, wenigstens einen Polymerisationsbeschleuniger und gegebenenfalls wenigstens einen Co-Polymerisationsbeschleuniger beinhaltet.

9. Paste nach einem der vorhergehenden Ansprüche, wobei das lösliche Polymer ausgewählt ist aus der Gruppe bestehend aus Poly(methacryl-säuremethylester), Poly-(methacrylsäureethylester), Poly-(methylmeth-acrylsäurepropylester), Poly(methacrylsäureisopropylester), Poly(methyl-methacrylat-co-methyl-acrylat), Poly(styrol-co-methyl-meth-acrylat) und einer Mischung aus mindestens zwei dieser Polymere.

10. Paste nach einem der vorhergehenden Ansprüche, wobei die Paste spätestens 15 Minuten nach ihrer Herstellung klebfrei nach ISO 5833 ist.

11. Kit, aufweisend eine Paste A und eine Paste B,
wobei
(a) Paste A
(a1) wenigstens ein radikalisch polymerisierbares Monomer,
(a2) wenigstens ein in (a1) lösliches Polymer, und
(a3) wenigstens einen Polymerisationsinitiator beinhaltet;
(b) Paste B
(b1) wenigstens ein radikalisch polymerisierbares Monomer,
(b2) wenigstens ein in (b1) lösliches Polymer, und
(b3) wenigstens einen Polymerisationsbeschleuniger beinhaltet;
und wobei mindestens eine der Pasten A und B als Komponente (a4) bzw. (b4) wenigstens einen in (a1) bzw. (b1) schwer- oder unlöslichen Füllstoff, wie in einem der Ansprüche 1 bis 5 definiert, beinhaltet.

12. Kit nach Anspruch 11, wobei das radikalisch polymerisierbare Monomer (a1) bzw. (b1) ein Methacrylsäureester ist.

13. Kit nach Anspruch 11 oder 12, wobei der wenigstens eine Polymerisationsinitiator (a3) ausgewählt ist aus der Gruppe der Barbiturate und der Peroxide.

14. Kit nach Anspruch 13 wobei der wenigstens eine Polymerisationsinitiator (a3) ausgewählt ist aus der Gruppe bestehend Cumol-hydroperoxid, 1,1,3,3-Tetramethylbutyl-hydroperoxid, t-Butyl-hydroperoxid, t-Amyl-hydroperoxid, Di-Isopropylbenzen-monohydroperoxid und einer Mischung aus mindestens zwei davon.

15. Kit nach einem der Ansprüche 11 bis 14, wobei die Paste A den wenigstens einen Polymerisationsinitiator (a3) in einer Menge in einem Bereich von 0,01 bis 10 Gew.-%, bezogen auf das Gesamtgewicht der Paste A, beinhaltet.

16. Kit nach einem der Ansprüche 11 bis 15, wobei der wenigstens eine Polymerisationsbeschleuniger (b3) wenigstens eine Schwermetallverbindung ist, die aus der Gruppe bestehend aus Schwermetallsalzen und Schwermetallkomplexen ausgewählt ist.

17. Kit nach Anspruch 16, wobei die Schwermetallverbindung ausgewählt ist aus der Gruppe bestehend aus Kupfer(II)-hydroxid, Kupfer(II)methacrylat, Kupfer(II)acetylacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

18. Kit nach Anspruch 16 oder 17, wobei die Paste B den Polymerisationsbeschleuniger (b3) in einer Menge in einem Bereich von 0,0005 bis 0,5 Gew.-%, bezogen auf das Gesamtgewicht der Paste B, beinhaltet.

19. Kit nach einem der Ansprüche 11 bis 15, wobei der wenigstens eine Polymerisationsbeschleuniger (b3) ausgewählt ist aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en, 1,5-Diazabicyclo(4.3.0)non-5-en, Phthalimid, Maleimid, Succinimid, Pyromelithsäurediimid und eine Mischung aus mindestens zwei davon.

20. Kit nach Anspruch 19, wobei die Paste B den Polymerisationsbeschleuniger (b3) in einer Menge in einem Bereich von 0,1 bis 10 Gew.-%, bezogen auf das Gesamtgewicht der Paste B, beinhaltet.

21. Kit nach einem der Ansprüche 11 bis 20, wobei die Paste B als Polymerisationsbeschleuniger (b3) eine Kombinationen aus Schwermetallsalzen und mindestens einem Vertreter aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)-non-5-en, Phthalimid, Maleimid, Succinimid und Pyromelithsäurediimid beinhaltet.

22. Kit nach einem der Ansprüche 11 bis 21, wobei die Paste A wenigstens ein Co-Polymerisationsbeschleuniger (a5) beinhaltet, der ausgewählt ist aus der Gruppe bestehend aus N,N-Dimethyl-p-toluidin, N,N-Bis-hydroxyethyl-p-toluidin, N,N-Dimethyl-anilin, 1,8-Diazabicyclo[5.4.0]-undec-7-en, 1,5-Diazabi-cyclo(4.3.0)non-5-en und einer Mischung aus mindestens zwei davon.

23. Kit nach Anspruch 22, wobei die Paste A den wenigstens einen Co-Polymerisationsbeschleuniger (a5) in einer Menge in einem Bereich von 0,1 bis 10 Gew.-%, bezogen auf das Gesamtgewicht der A, beinhaltet.

24. Kit nach einem der Ansprüche 11 bis 23, wobei die Paste A und die Paste B das wenigstens eine radikalisch polymerisierbare Monomer (a1) bzw. (b1) in einer Menge in einem Bereich von 15 bis 85 Gew.-%, jeweils bezogen auf das Gesamtgewicht der Paste A bzw. der Paste B, beinhalten.

25. Kit nach einem der Ansprüche 11 bis 24, wobei das in (a1) bzw. (b1) lösliche Polymer (a3) bzw. (b3) ausgewählt ist aus der Gruppe bestehend aus Poly(methacrylsäuremethylester), Poly(methacrylsäureethylester), Poly-(methylmethacrylsäurepropylester), Poly(methacrylsäureisopropyl-ester), Poly(methylmeth-acrylat-co-methylacrylat), Poly(styrol-co-methyl-meth-acrylat) und einer Mischung aus mindestens zwei dieser Polymere.

26. Kit nach einem der Ansprüche 11 bis 25, wobei Paste A 20 bis 70 Gew.-%, bezogen auf das Gesamtgewicht der Paste A, eines in den Ansprüchen 1 bis 5 definierten Füllstoffs (a4) und Paste B weniger als 5 Gew.-%, bezogen auf das Gesamtgewicht der Paste B, eines in den Ansprüchen 1 bis 5 definierten Füllstoffs (b4) beinhaltet.

27. Kit nach Anspruch 26, wobei Paste A das in (a1) lösliche Polymer (a2) in einer Menge in einem Bereich von 1 bis 25 Gew.-%, bezogen auf das Gesamtgewicht der Paste A, und Paste B das in (b1) lösliche Polymer (b2) in einer Menge in einem Bereich von 25 bis 85 Gew.-%, bezogen auf das Gesamtgewicht der Paste B, beinhaltet.

28. Kit nach einem der Ansprüche 11 bis 25, wobei Paste A 15 bis 85 Gew.-%, bezogen auf das Gesamtgewicht der Paste A, eines in den Ansprüchen 1 bis 5 definierten Füllstoffs (a4) und Paste B 15 bis 85 Gew.-%, bezogen auf das Gesamtgewicht der Paste B, eines in den Ansprüchen 1 bis 5 definierten Füllstoffs (b4) beinhaltet.

29. Kit nach Anspruch 28, wobei Paste A das in (a1) lösliche Polymer (a2) in einer Menge in einem Bereich von 5 bis 50 Gew.-%, bezogen auf das Gesamtgewicht der Paste A, und/oder Paste B das in (b1) lösliches Polymer (b2) in einer Menge in einem Bereich von 5 bis 50 Gew.-%, bezogen auf das Gesamtgewicht der Paste B, beinhaltet.

30. Kit nach einem der Ansprüche 11 bis 29, wobei die Pasten A und/oder B den in den Ansprüchen 1 bis 5 definierten Füllstoff (a4) bzw. (b4) in einer solchen Menge enthalten, das in der durch Vermischen der Pasten A und B hergestellten Paste C der Füllstoff in einer Menge in einem Bereich von 0,5 bis 25,0 Gew.-% enthalten ist.

31. Verwendung einer Paste gemäß einem der Ansprüche 1 bis 10, oder einer Paste, hergestellt aus dem Kit gemäß einem der Ansprüche 11 bis 30, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.

32. Formkörper, erhältlich durch die Polymerisation einer Paste gemäß einem der Ansprüche 1 bis 10, oder einer Paste hergestellt aus dem in den Ansprüchen 11 bis 30 definierten Kit.
